Europäisches Patentamt

European Patent Office

Office européen des brevets

(11) Veröffentlichungsnummer: **0 081 724**
**B1**

# (12) EUROPÄISCHE PATENTSCHRIFT

(45) Veröffentlichungstag der Patentschrift:
**12.03.86**

(21) Anmeldenummer: **82110953.5**

(22) Anmeldetag: **26.11.82**

(51) Int. Cl.⁴: **A 61 M 25/00,** A 61 M 1/28

(54) **Intraperitonealkatheter.**

(30) Priorität: **27.11.81 DE 3147206**
**02.12.81 DE 8135151 U**

(43) Veröffentlichungstag der Anmeldung:
**22.06.83 Patentblatt 83/25**

(45) Bekanntmachung des Hinweises auf die Patenterteilung:
**12.03.86 Patentblatt 86/11**

(84) Benannte Vertragsstaaten:
**AT BE CH DE FR GB IT LI LU NL SE**

(56) Entgegenhaltungen:
**DE - A - 2 149 041**
**US - A - 3 663 965**
**US - A - 3 853 126**
**US - A - 4 184 497**

(73) Patentinhaber: **Fresenius AG, Gluckensteinweg 5,
D-6380 Bad Homburg (DE)**

(72) Erfinder: **Bartz, Volker, Ludwig-Richter-Strasse 24,
D-6300 Giessen (DE)**
Erfinder: **Affeld, Klaus, Prof., Niebuhrstrasse 11a,
D-1000 Berlin 12 (DE)**
Erfinder: **Becker, Helmut, Dr., Bundesplatz 1,
D-1000 Berlin 31 (DE)**
Erfinder: **Krautzberger, Wolfgang, Prof.,
Weitselberweg 38a, D-7951 Elchingen-1 (DE)**
Erfinder: **Schurig, Randolf, Dr., Bonhoefer Ufer 18,
D-1000 Berlin 10 (DE)**
Erfinder: **Grosse-Siestrup, Christian, Dr.,
Rückerstrasse 8a, D-1000 Berlin 41 (DE)**

(74) Vertreter: **KUHNEN & WACKER Patentanwaltsbüro,
Schneggstrasse 3-5 Postfach 1729, D-8050 Freising (DE)**

ACTORUM AG

## Beschreibung

Die Erfindung betrifft einen Peritonealkatheter gemäss dem Obergegriff des Anspruchs 1.

Peritonealkatheter der eingangs erwähnten Art werden insbesondere bei der Peritonealdialyse von nierengeschädigten Patienten eingesetzt. Hierzu wird der Katheter im Unterleib des Patienten implantiert, wobei der Katheterschlauch durch eine Öffnung in der Epidermis in den Hautschichtenbereich eingeführt, dort seitlich versetzt abgebogen und anschliessend durch die Peritonealwand in den Peritonealraum geführt wird. Demzufolge weist der bekannte Katheter ein extrakorporales Katheterstück, ein in den Hautschichten befindliches weiteres Katheterstück und schliesslich ein im Peritonealraum angeordnetes drittes Katheterstück auf, die zusammen den Peritonealkatheter bilden.

Ein derartiger bekannter Katheter ist beispielsweise in der GB-A-1307055 beschrieben. Hieraus ist ersichtlich, dass derart seitlich versetzt implantierte Katheter aufgrund ihrer elastischen Eigenschaften unter Spannung stehen, was zu Problemen beim Einwachsen des Katheters in die Hautschichten führt. Infolgedessen ist der bekannte Katheter mit einer gewebefreundlichen Beschichtung sowie einem Reservoir mit einer antiseptischen Flüssigkeit versehen, das den Katheter umgibt. Diese antiseptische Flüssigkeit soll solange die Einschleppung von Keimen verhindern, bis die den Katheter umgebende Haut in die gewebefreundliche Schicht eingewachsen ist, wodurch das Eindringen von Keimen infolge Versetzung des Katheters verhindert wird.

Abgesehen davon, dass eine derartige antiseptische Flüssigkeit regelmässig zu Irritationen der Haut führt, können bei einer derartigen Anordnung immer noch Keime in die Hautschichten und in den Peritonealraum selbst eindringen, da die vom Katheter selbst ausgeübten Zugspannungen und Oberflächenscherkräfte nicht wirksam aufgefangen werden können.

Infolgedessen besteht ein Bedürfnis nach einem Peritonealkatheter, der diese Probleme löst.

Die US-A-4184497 beschreibt einen Peritonealkatheter, der völlig innerhalb des Patienten implantiert ist, also kein aus dem Patienten ragendes extrakorporales Katheterschlauchstück aufweist. Daher treten bei diesem Katheter nicht die Kontaminationsprobleme auf, die bei den üblichen Peritonealkathetern – wie dies vorstehend beschrieben ist – auftreten. Andererseits muss die Bauchwand jeweils mit der Kanüle einer Spritze durchstochen werden, wenn eine extrakorporale Verbindung mit dem Peritonealkatheter hergestellt werden soll. Dies ist jedoch einerseits schmerzhaft und andererseits kontaminationsgefährdend, da die Kanüle beim Durchstechen der Haut mit Keimen verunreinigt werden kann.

Ein weiterer Katheter ist aus der US-A-3663965 bekannt, der im wesentlichen dem Katheter gemäss vorstehender GB-A-1307055 gleicht, jedoch mit einer hautverträglichen Scheibe umgeben ist, die in die Hautschichten implantiert werden soll.

Diese Scheibe soll mit den Hautschichten verwachsen und somit die am Katheter auftretenden Zugspannungen aufnehmen. Der Katheter selbst bleibt jedoch unverändert, erzeugt also bei der zeitlich versetzten Implantation die vorstehend erwähnten Spannungen, die von der Scheibe aufgefangen werden sollen. Auch dieser Vorschlag zur Lösung der Spannungsprobleme bei den üblichen Kathetern ist noch nicht zufriedenstellend, da zumindest während der Einwachsphase immer noch eine erhebliche Kontaminationsgefahr infolge Riss- und Spaltenbildung besteht.

Der Erfindung liegt daher die Aufgabe zugrunde, den Peritonealkatheter gemäss dem Oberbegriff des Anspruchs 1 so fortzubilden, dass die nach der Implantation auftretende Kontaminationsgefahr erheblich verringert bzw. beseitigt wird.

Die Lösung der Aufgabe erfolgt durch die kennzeichnenden Merkmale des Anspruchs 1.

Der erfindungsgemässe Peritonealkatheter lässt zunächst eine Implantation zu, bei der bereits kurz nach dem Implantieren kein Einschleppen von Keimen mehr zu erwarten ist. Dies ist insbesondere darauf zurückzuführen, dass an der Austrittsöffnung des Katheterrohrs aus der Epidermis des Patienten keinerlei Zug- oder Scherspannungen auftreten, wie dies bei einem üblichen Katheter zu beobachten ist. Demzufolge kann der erfindungsgemässe Peritonealkatheter erfolgreich bei der Tunnelimplantation eingesetzt werden, bei der der Katheter seitlich abknickt und quer zur Bauchwand verläuft, da vom Katheter selbst im wesentlichen keine Spannungen mehr auf die umliegenden Hautbereiche übertragen werden. Die spannungsfrei vorgebogene Form des Katheters gewährleistet also, dass die Riss- und Spaltenbildung in der Epidermis verhindert wird, die ansonsten das Einschleppen von Keimen begünstigen würde.

In einer weiteren bevorzugten Ausführungsform ist das aus dem Körper ragende Stück des Katheters ebenfalls spannungsfrei abgebogen und verläuft im wesentlichen parallel zur Oberfläche des Körpers des Patienten. Somit kann also die vom Patienten getragene Kleidung keinerlei Spannung mehr auf den Katheter ausüben, mit der Folge, dass insgesamt gesehen der Katheter im wesentlichen spannungsfrei im und am Körper des Patienten getragen werden kann.

Der erfindungsgemässe Intraperitonealkatheter zeichnet sich zunächst dadurch aus, dass er praktisch spannungsfrei unter der Bauchhaut verlegt werden kann, dass also im verlegten Zustand keine Spannungen mehr auftreten. Somit ziehen die Katheterteile, die durch die Körperöffnungen geführt worden sind, nicht mehr an den Umfangsrändern dieser Öffnungen und verformen diese nicht. Vielmehr sind sie durch diese Öffnungen spannungsfrei geführt, so dass sich die Umfangsränder eng an den Katheter anlegen und diesen abdichten können. Hierdurch werden im wesentlichen sämtliche die Kontamination unterstützenden Durchtrittsöffnungen beseitigt, so dass von dem erfindungsgemässen Katheter praktisch keine Kontaminationsgefahren mehr zu erwarten sind.

Weiterhin weist der erfindungsgemässe Katheter eine relativ hohe Elastizitätsreserve auf, d. h. er lässt sich infolge seiner spannungsfreien Form in Längsrichtung erheblich spannen, was insbesondere bei Relativbewegungen zwischen Bauchhaut und Peritoneum von Bedeutung ist. Die dabei zwangsläufig an den Durchtrittsöffnungen auftretenden Zugkräfte sind dabei so gering, dass sie ohne nachteilige Folgen bleiben.

In einer speziellen Ausführungsform ist dieser Bereich wellenförmig ausgestaltet und lässt sich infolge der hohen Flexibilität des Katheters um ein Mehrfaches hinsichtlich seiner ungespannten Länge verlängern, so dass praktisch keine nennenswerten Spannungskräfte an den Körperöffnungen mehr auftreten.

Besonders hervorzuheben ist jedoch die Tatsache, dass durch den erfindungsgemässen Katheter ohne Schwierigkeiten ein gerader Mandrin, beispielsweise aus Edelstahl, hindurchgesteckt werden kann, so dass die vorstehend erwähnte Implantation problemlos durchgeführt werden kann. Nach dem Herausziehen des Mandrins kehrt der Katheter in seine urspüngliche gebogene Form zurück und verbleibt in dieser. Er wird nunmehr durch den Kanal und die vorbereitete zweite Körperöffnung gezogen und befindet sich dann in seinem endgültigen implantierten Zustand.

Um seinen sicheren Sitz an den Körperöffnungen bzw. im Kanal zu gewährleisten, ist in einer weiteren speziellen Ausführungsform wenigstens eine Scheibe vorgesehen, deren Mittelpunkt von dem Katheter durchsetzt wird. Diese Scheibe soll wie die bekannte Manschette von der Haut um- bzw. durchwachsen werden, damit ein fester und sicherer Sitz des Katheters an den Körperöffnungen bzw. im Kanal sichergestellt wird.

In einer weiteren speziellen Ausführungsform ist der aus dem Körper des Patienten ragende Katheterteil ebenfalls abgebogen und liegt praktisch am Körper des Patienten an. Bei dieser Ausführungsform treten keine durch den Katheter hervorgerufenen Spannungsprobleme mehr auf, die sich negativ auf die in der Bauchwand befindliche Öffnung auswirken könnten. Vielmehr bleibt dieser Bereich spannungsfrei. Ausserdem lässt sich der Katheter sehr gut zwischen der Körperoberfläche und den Bekleidungsstücken unterbringen, was eine angenehme Trageweise für den Patienten darstellt.

Bei dem bekannten Intraperitonealkatheter ging man grundsätzlich davon aus, dass die auftretenden Kontaminationen im wesentlichen unvermeidlich sind und somit ein Austausch des Katheters bzw. ein erneutes Setzen des Katheters von Zeit zu Zeit vorzunehmen ist. Überraschenderweise wurde nunmehr bei dem erfindungsgemässen Intraperitonealkatheter festgestellt, dass die auftretenden Unverträglichkeiten bzw. Kontaminationen im wesentlichen auf die vom Katheter ausgeübten Spannungen zurückzuführen sind. Diese Probleme sind nämlich beim erfindungsgemässen Katheter im wesentlichen beseitigt, was an sich unerwartet war, da die Fachwelt von einem systemimmanenten Fehler ausging.

Weitere Einzelheiten, Merkmale und Vorteile der Erfindung ergeben sich aus der nachfolgenden Beschreibung unter Bezugnahme auf die Zeichnung.

Es zeigen:

Fig. 1 den erfindungsgemässen Intraperitonealkatheter in einer ersten Ausführungsform im Längsschnitt, wobei lediglich der durch die Haut führende Bereich vergrössert gezeigt ist

Fig. 2 eine weitere Ausführungsform eines erfindungsgemässen Katheters in der gleichen Darstellung gemäss Fig. 1

Fig. 3 eine dritte Ausführungsform des erfindungsgemässen Katheters in der Darstellung wie Fig. 1

Fig. 4 eine vierte Ausführungsform des erfindungsgemässen Katheters in der Darstellungsweise gemäss Fig. 1

Fig. 5 eine fünfte Ausführungsform des erfindungsgemässen Katheters in der Darstellungsweise gemäss Fig. 1

Fig. 6 eine sechste Ausführungsform in der Darstellungsweise gemäss Fig. 1 und

Fig. 7 eine weitere Ausführungsform in der Darstellungsweise gemäss Fig. 1.

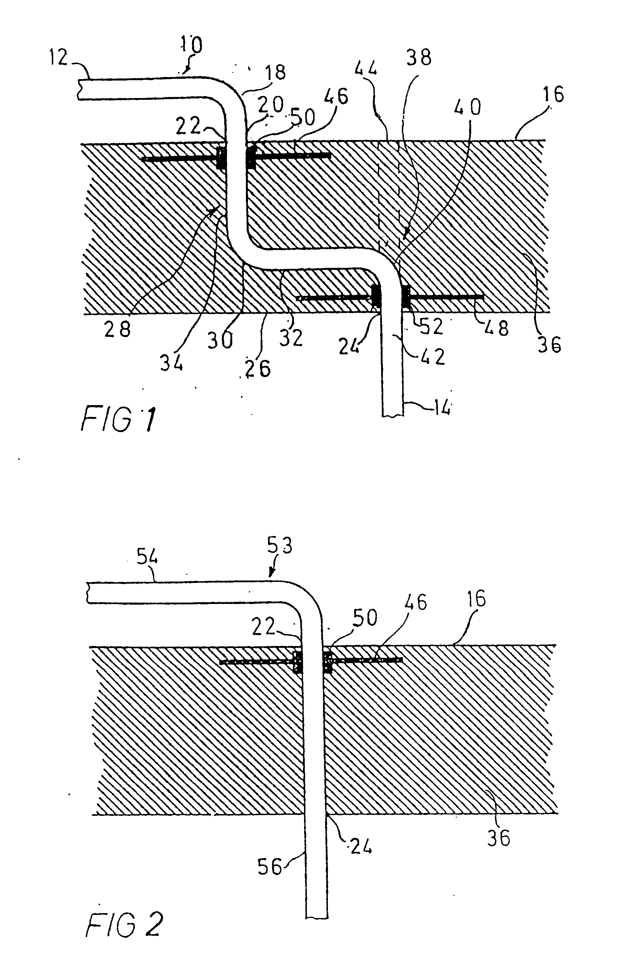

In Fig. 1 ist die erste Ausführungsform des Katheters 10 gezeigt, bei dem aus Gründen der Übersichtlichkeit die hier beiliegenden Endstücke weggelassen sind. Es führen also die Bereiche 12 und 14 zu den Enden des Katheters 10, die einen Anschluss für einen Konnektor bzw. Drainageöffnungen für den erleichterten Ab- und Zulauf der Dialyseflüssigkeit im Peritonealraum aufweisen.

Der Katheter 10 weist gemäss der ersten Ausführungsform eine in der Nähe der Körperhaut 16 des Patienten gebogene Form 18 vor dem Körper des Patienten auf, die vorteilhafterweise derart ausgestaltet ist, dass der sich an die gebogene Form 18 anschliessende Bereich 20 in seiner Längsachse senkrecht auf die Körperhaut 16 trifft.

Dieser Bereich 20 des Katheters durchsetzt die erste Öffnung 22 im Körper des Patienten, die der Chirurg als zweite Körperöffnung geöffnet hat. An die Öffnung 22 schliesst sich bis zur zweiten Öffnung 24 in der Wand 26 des Peritonealraums der durch die Hautschichten führende Bereich oder nachstehend Tunnelbereich 28 genannte Bereich des Katheters 10 an.

Der Tunnelbereich 28 gemäss Fig. 1 ist in dieser bevorzugten Ausführungsform etwa in der Mitte im wesentlichen rechtwinklig abgeknickt, weist also eine seitliche abgebogene Form 30 auf. Diese abgebogene Form 30, die bereits fertigungsseitig festgelegt ist, liegt im implantierten Zustand im wesentlichen spannungsfrei vor, so dass der Katheter 10 in seinem durch die Öffnungen 22 und 24 führenden Bereichen keine Spannungen aufweist. Hierdurch wird sichergestellt, dass keine Keime durch infolge von Spannungen auftretende Zwischenräume zwischen der Öffnung 22 und 24 und dem Katheter 10 eindringen können. Sollte dennoch eine Infizierung an oder um die Stelle 22 erfolgen, so kann diese infolge der abgebogenen Form 30 des Katheters nicht zu der Öffnung 24 durchdringen.

In einer weiteren bevorzugten Ausführungsform sind die Schenkel 32 und 34, die sich von der abgebogenen Form 30 erstrecken im wesentlichen gleich lang. Dies muss andererseits jedoch nicht zwangsläufig vorliegen, so dass auch der Schenkel 32, sofern erforderlich, länger sein kann als der Schenkel 34. Üblicherweise beträgt die Länge der Schenkel 32 und 34 etwa eins bis vier, vorzugsweise ca. zwei cm.

Weiterhin müssen die Längsachsen der Schenkel 32 und 34 nicht zwangsläufig im wesentlichen rechtwinklig aufeinanderstehen; so können sie auch in anderen Winkeln zueinander vorliegen, sofern dies von den Hautformationen gefordert wird. So wird man bei Patienten mit dickeren Bauchdecken im wesentlichen Katheter 10 mit rechtwinklig abgebogenen Formen 30 einsetzen, während man bei Patienten mit dünnen Bauchdecken, beispielsweise Kindern, zu abgebogenen Formen 30 übergehen wird, deren Öffnungswinkel bis zu 150° aufgeweitet sein kann. Andererseits kann jedoch aber auch der rechtwinklige Zustand beibehalten werden, wenn die Schenkel 32 und 34 entsprechend verlängert bzw. verkürzt werden.

Da sich der Schenkel 32 des Katheters 10 im wesentlichen parallel zur Wand 26 des Peritonealraumes durch die Hautschicht 36 erstreckt, die zwischen der Wand 26 des Peritonealraums und der Körperhaut 16 gebildet wird, weist der Katheter 10 in dem sich an den Schenkel 32 anschliessenden Bereich 38 eine erneute abgebogene Form 40 auf, die vorteilhafterweise so ausgestattet ist, dass sich das daran erstreckende Katheterteil 42 durch die Wand 26 des Peritonealraumes und die Öffnung 24 erstreckt.

Der zwischen dem Schenkel 34 und dem Katheterteil 42 gebildete Winkel beträgt im wesentlichen 90° kann jedoch aber auch im Bedarfsfall grösser oder kleiner sein, sofern die Spannungsfreiheit im Bereich der Öffnung 24 sichergestellt ist.

Üblicherweise wird dieser Katheter 10 dadurch eingesetzt, dass zunächst die Körperhaut 16 bei 44 geöffnet wird, wobei diese Öffnung 44 mit der Öffnung 24 fluchtet. Nachdem der Katheter 10 auf einem geraden Mandrin aufgesteckt worden ist, was infolge seiner Flexibilität problemlos ist, wird dieser Mandrin mit dem Katheter 10 durch die Öffnungen 44 und 24 durchgesteckt. Anschliessend wird der Mandrin entfernt und es wird das extrakorporale Ende des Katheters 10 durch die Öffnung 44 in die Hautschicht 36 gezogen und von dort durch die Öffnung 22 hindurchgezogen.

Dieser Katheter 10 kann infolge seiner Spannungsfreiheit ohne zusätzliche Hilfsmittel einwachsen. Vorteilhafterweise ist jedoch wenigstens ein Verankerungsmittel im Tunnelbereich vorgesehen, um ein unabsichtliches Herausziehen des Katheters 10 zu verhindern. In der in Fig. 1 gezeigten bevorzugten Ausführungsform sind in der Nähe der Körperöffnungen 22 und 24 zwei Scheiben 46 und 48 vorgesehen, deren Mittelpunkt von dem Schenkel 34 bzw. dem Katheterteil 42 durchsetzt wird. Die Form dieser Scheiben 46 und 48 ist unkritisch und ist im allgemeinen kreisförmig. Sie weist üblicherweise einen Durchmesser von eins bis vier, vorzugsweise etwa 1,5–2,5 cm auf.

Diese Scheiben 46 und 48 sind natürlich aus einem körperfreundlichen Material gefertigt, das als Kunststoff oder als Metall, wie Titan vorliegen kann. Zu einsetzbaren Kunststoffen gehören Produkte, die unter der Bezeichnung «Dacron» im Handel vertrieben werden. Die Oberfläche dieser Scheiben 46 und 48 kann mit diesem Kunststoffmaterial beschichtet sein, während der Kern aus einem anderen Material, beispielsweise Silikon, gefertigt sein kann. Diese Kernschicht kann im Bereich des Katheters 10 durch eine Manschette 50 bzw. 52 stabilisiert sein, wobei diese Manschette 50 und 52 sowohl die Halterung der Scheibe am Katheter 10 selbst verbessern als auch eine gleichmässige Flexibilität der Scheiben 46 und 48 gewährleisten.

Durch diese Scheiben 46 und 48, die von den jeweiligen Hautschichten be- und/oder durchwachsen werden können, wird einerseits ein Eindringen von Keimen in die Bauchhöhle vermieden und andererseits ein Herausfliessen der Dialyselösung im Implantationskanal verhindert. Durch die spezielle Ausgestaltung dieser Scheiben in Verbindung mit dem Katheter 10 wird eine mechanische Belastung der eingewachsenen Scheiben 46 und 48 im wesentlichen vermieden bzw. kann dieselbe durch diese Scheiben 46 und 48 und durch die angewachsenen Hautbereiche aufgefangen werden.

In Fig. 2 ist eine zweite Ausführungsform des Katheters 10 gezeigt, bei dem gleiche Bezugszeichen gleiche Teile wie bei dem Katheter gemäss Fig. 1 bezeichnen. Dieser Katheter 53 durchsetzt die beiden Öffnungen 22 und 24 fluchtend, so dass der unmittelbar vor der Körperhaut 16 liegende und dazu parallel ausgerichtete Katheterast 54 und der Katheterast 56 im wesentlichen rechtwinklig aufeinanderstehen. In der Nähe der Öffnung 22 ist wiederum eine Scheibe 46 einschliesslich der dazugehörigen Manschette 50 vorteilhafterweise angeordnet.

Der in Fig. 2 gezeigte Katheter 53 wird vorteilhafterweise entweder als Kurzzeitkatheter oder bei Patienten mit dünnen Bauchdecken eingesetzt, bei denen die Körperhaut 16 und die Wand 26 des Peritonealraumes eng beieinanderliegen, so dass die Scheibe 46 praktisch zwischen beiden verwachsen kann und somit ein Eindringen von Keimen und/oder Durchdringen von Dialyseflüssigkeit durch die Scheibe 46 verhindert werden kann.

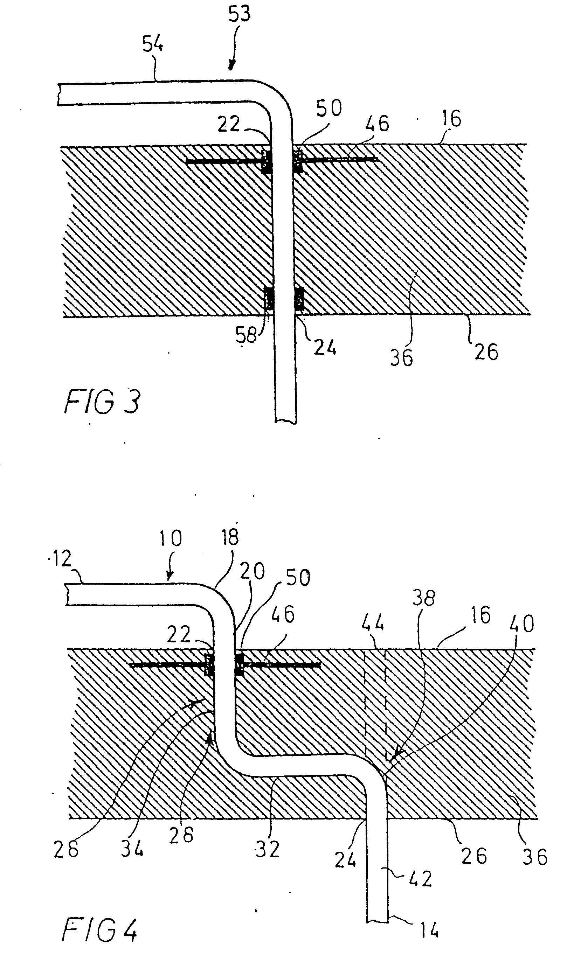

In Fig. 3 ist eine Abwandlung der in Fig. 2 gezeigten Ausführungsform gezeigt. Gegenüber der letztgenannten Ausführungsform tritt der in Fig. 3 gezeigten Ausführungsform des Katheters 53 eine Manschette 58 hinzu, die in der Nähe der Öffnung 24 angeordnet ist. Diese Manschette 58 soll ein besseres Verwachsen der um die Öffnung 24 befindlichen Hautbereiche mit dem Katheter 53 gewährleisten und somit insbesondere einen Durchtritt von Dialyselösung in diese Hautbereiche verhindern.

Die in Fig. 4 gezeigte Ausführungsform, die im wesentlichen der in Fig. 1 gezeigten Ausführungsform des Katheters 10 ähnelt, unterscheidet sich von dieser lediglich dadurch, dass die Scheibe 48 weggelassen worden ist. Hierdurch wird die Implantation und Explantation des erfindungsgemässen Katheters 10 erheblich erleichtert. Weiterhin kann diese Ausführungsform häufig ausreichen, um die gewünschte Eigenschaft des Katheters 10, nämlich die Veränderung von Kontamination durch Keime und die Abdichtung gegen das Eindringen von Dialyseflüssigkeit, herzustellen. Somit stellt diese Ausführungsform neben der in Fig. 1 gezeigten Ausführungsform eine besonders vorteilhafte Variante dar.

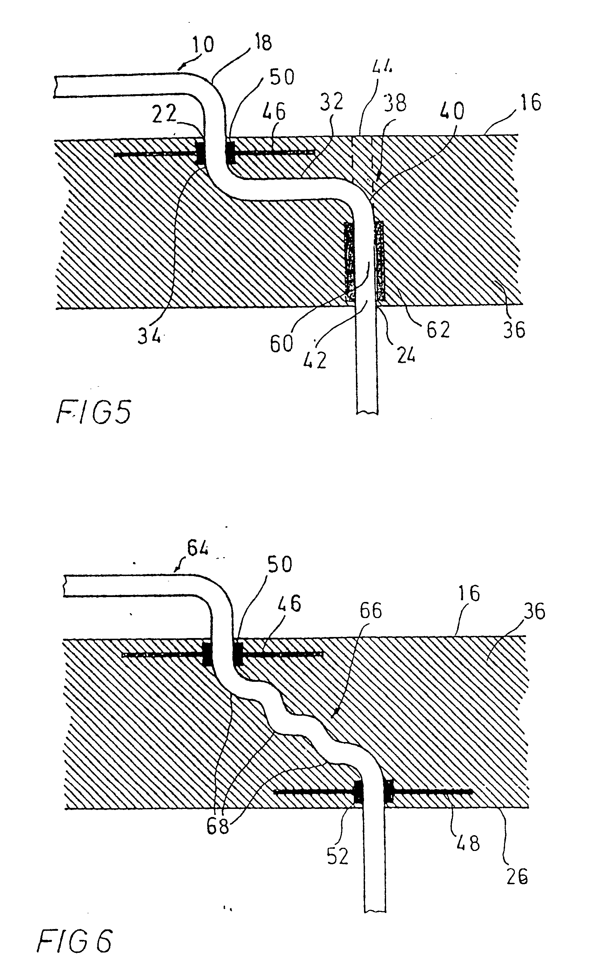

In Fig. 5 ist eine weitere Variation der in Fig. 1 gezeigten Ausführungsform gezeigt. Hieraus ist ersichtlich, dass der Katheter 10 einen besonders kurzen Schenkel 34 und demgegenüber einen erheblich verlängerten Schenkel 32 aufweist. Zusätzlich ist das Katheterteil 43 durch die Öffnung 24 in die Hautschichten 36 zurückgeschoben, was ebenfalls eine Zurückziehung des Bereichs 38 in den mittleren Teil der Hautschicht 36 bewirkt. Der sich an die abgerundete Form 40 anschliessende Katheterbereich 60 des Katheterteils 42 ist praktisch vollständig mit der zu einem Kragen 62 verlängerten Manschette 52 umgeben, der einerseits als Abstandshalter für den abgebogenen Bereich 40 von der Öffnung 24 und andererseits als Verankerungshilfe für den Katheter in der Bauchhaut dient. Eine solche Ausführungsform wird dort eingesetzt, wo eine scheibenförmige Ausführung keine genaue Verankerung mehr gewährleistet und eine kragenförmige, verlängerte Verankerungshilfe angebracht ist.

Eine weitere Ausführungsform ist in Fig. 6 gezeigt. In dieser Ausführungsform, die einen Katheter 64 zeigt, ist der zwischen den Hautschichten 16 und 26 befindliche Bereich 66 mit mehreren seitlichen Abbiegungen 68 versehen, wobei diese seitlichen Abbiegungen 68 vorzugsweise wellenförmig ausgestaltet sind und die durch die Haut tretenden Bereiche des Katheters 64 miteinander verbinden. Vorteilhafterweise sind an den durch die Haut im wesentlichen senkrecht tretenden Bereichen des Katheters 64 die Scheiben 46 und 48 angebracht, von denen sich die seitlichen Abbiegungen 68 erstrecken. Diese Abbiegungen 68, die vorteilhafterweise mäanderförmig, spiralig und/ oder schraubenförmig hinsichtlich der Kathetermittelachse ausgebildet sein können, gestatten eine besonders leichte Verschieblichkeit der Scheiben 64 und 68 gegeneinander und ermöglichen hierdurch einen sehr guten Ausgleich von Relativbewegungen der mit 16 bezeichneten Epidermis und der Wand 26 des Peritonealraumes.

Ein erfindungsgemässer Katheter 10, 53 oder 64 wird bereits werkseitig in der gewünschten Form gefertigt und liegt somit im Einsatz spannungsfrei vor. Da vorteilhafterweise polymere Materialien, insbesondere Silikonprodukte, zur Herstellung der erfindungsgemässen Katheter eingesetzt werden, werden die üblichen in der Kunststofftechnik eingesetzten Verarbeitungsmethoden angewandt. So wird beispielsweise ein Silikonmaterial zu einem Schlauch verarbeitet und in einer entsprechend geformten Matrize zu dem erfindungsgemässen Katheter ausgehärtet, wobei das Anbringen der Scheiben 46 und 48 entweder direkt in der Matrize oder anschliessend erfolgen kann.

Hinzuzufügen ist, dass es sowohl aus Fertigungsgründen als auch aus Anwendungsgründen vorteilhaft ist, wenn durch den gesamten Katheter eine Ebene gelegt werden kann. Sollten allerdings die seitlichen Abbiegungen 68 in ihrer Wellenform zu stark sein, so kann es angebracht sein, dass die durch diese Welle gelegte Ebene senkrecht auf der Katheterebene steht.

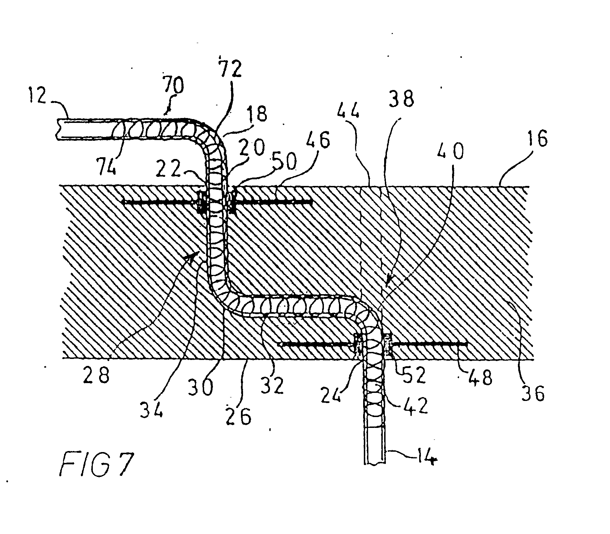

In Fig. 7 ist eine weitere Ausführungsform des erfindungsgemässen Intraperitonealkatheters 70 dargestellt, der sowohl aus fertigungstechnischen Gründen als auch wegen seiner leichten Einsetzbarkeit besonders bevorzugt ist.

Dieser Katheter 70 weist in seiner Wandung 72 ein diese Wandung versteifendes Element auf, das einerseits dem Katheter 70 eine stabilere Form völlige Spannungsfreiheit verleiht und andererseits bleibend verformbare Eigenschaften aufweist.

Ein derartiges versteifendes Element kann beispielsweise in Form eines oder mehrerer Drähte vorliegen, die in der Wand 72 des Katheters 70 eingebettet sind. Es ist insbesondere vorteilhaft, wenn ein derartiger Draht wendelförmig in die Wand 72 eingebettet ist und somit die Form der in Fig. 7 gezeigten Spirale 74 aufweist.

Diese Spirale 74 kann in dem Katheterbereich vorliegen, der unmittelbar an die Bauchwand angrenzt und durch diese geführt wird. Andererseits kann jedoch aber auch diese Spirale 74 vollständig durch den gesamten Katheter 70 geführt sein, wobei eine derartige Führung voraussetzt, dass der Katheter 70 noch ausreichend flexibel ist.

Letztere Ausführungsform wird man insbesondere dort wählen, wo der Katheter bereits werksseitig in seiner endgültig gekrümmten Form vorgefertigt wird. In diesem Fall reichen bereits geringe Rückstellkräfte dieses versteifenden Elements aus, da der Katheter selbst bereits spannungsfrei ist.

Andererseits kann jedoch aber auch der Katheter 70 in gestreckter, d.h. gerader Form vorgefertigt sein und dieses versteifende Element aufweisen. Der Katheter wird dann beim Einsetzen entsprechend der gewünschten Führung durch die Bauchwand vom Chirurgen gebogen und verbleibt in dieser endgültig gebogenen Form im Körper des Patienten. Da die Wand 72 des Katheters 70 infolge dieser Abbiegungen Spannungen aufweist, muss sichergestellt sein, dass das versteifende Element diese im Knickbereich auftretende Spannungen derart aufnimmt, dass er im wesentlichen bleibend verformt vorliegt. Bei dieser Ausführungsform wird man somit ein elastisches Element mit geringerer Elastizität einsetzen.

## Patentansprüche

1. Flexibler Peritonealkatheter für die dauerhafte Implantation bei der Peritonealdialyse, der ein mit einem Konnektor verbindbares distales Ende, einen durch die Bauchwand des Patienten führbaren Bereich und ein in die Bauchhöhle einführbares proximales Ende aufweist und mindestens eine Krümmung besitzt, dadurch gekennzeichnet, dass die Krümmung(en) in dem Katheterbereich, der vor und/oder in der Bauchwand angebracht und/oder implantiert ist, derartig spannungsfrei vorgefertigt ist (sind), dass die Krümmung(en) bei der Implantation in den gestreckten Zustand überführbar ist (sind) und aufgrund ihrer flexiblen Eigenschaft nach der Implantation in den vorgefertigt gekrümmten Zustand selbsttätig zurückkehrt (zurückkehren).

2. Katheter nach Anspruch 1, gekennzeichnet durch zwei in der Bauchwand liegende Schenkel (32; 34), die im wesentlichen rechtwinklig zueinander angeordnet vorliegen.

3. Katheter nach Anspruch 1 oder 2, dadurch gekennzeichnet, dass der unmittelbar vor der Bauchwand liegende Bereich des Katheters (10, 53, 64) im wesentlichen rechtwinklig abgebogen vorliegt.

4. Katheter nach einem der Ansprüche 1–3, dadurch gekennzeichnet, dass der vor der Peritonealwand angeordnete Bereich (38) des Katheters (10, 64) im wesentlichen rechtwinklig abgebogen ist.

5. Katheter nach einem der Ansprüche 1–4, dadurch gekennzeichnet, dass die Schenkel (32, 34) im wesentlichen gleich lang sind.

6. Katheter nach Anspruch 1–3 oder 5, gekennzeichnet durch eine Vielzahl von wellenförmigen Abbiegungen (68) in dem durch die Bauchwand führenden Bereich.

7. Katheter nach einem der Ansprüche 1–6, gekennzeichnet durch wenigstens eine ihn umgebende Scheibe (46, 48) aus körperfreundlichem Material.

8. Katheter nach Anspruch 7, dadurch gekennzeichnet, dass die Scheibe (46, 48) eine unmittelbar den Katheter (10, 53, 64) umgebende Manschette (50, 52) aufweist.

9. Katheter nach Anspruch 7 oder 8, dadurch gekennzeichnet, dass die Scheibe (46, 48) einen Kernbereich aufweist, der mit einem körperfreundlichen Material beschichtet ist, wobei das Material des Kernbereichs und der Manschette (50, 52) identisch sind.

10. Katheter nach einem der Ansprüche 7–9, dadurch gekennzeichnet, dass das körperfreundliche Material ein Polyestergewebe auf der Basis von Polyethylenterphthalat (Dacron) und/oder Titan ist.

11. Katheter nach einem der Ansprüche 7–10, dadurch gekennzeichnet, dass er zwei Scheiben (46, 48) aufweist.

12. Katheter nach einem der Ansprüche 7–11, dadurch gekennzeichnet, dass er eine Scheibe (46, 48) und eine Manschette (58, 62) aufweist.

13. Katheter nach einem der Ansprüche 1–12, dadurch gekennzeichnet, dass er aus einem Silikonmaterial besteht.

14. Katheter nach einem der Ansprüche 1–13, dadurch gekennzeichnet, dass durch ihn eine Ebene legbar ist.

15. Katheter nach einem der Ansprüche 1–14, dadurch gekennzeichnet, dass er wenigstens ein bleibend verformbares Element in der Wand (72) aufweist.

16. Katheter nach Anspruch 15, dadurch gekennzeichnet, dass er ein oder mehrere in der Katheterlängsachse verlaufende Drähte aufweist.

17. Katheter nach Anspruch 15 oder 16, dadurch gekennzeichnet, dass der Draht die Form einer Spirale (74) aufweist.

18. Katheter nach einem der Ansprüche 15–17, dadurch gekennzeichnet, dass das bleibend verformbare Element in dem durch die Bauchwand führbaren Bereich des Katheters (7) vorgesehen ist.

19. Katheter nach einem der Ansprüche 15–18, dadurch gekennzeichnet, dass er ein bleibend verformbares Element mit hoher Elastizität aufweist und abgebogen vorgefertigt ist.

20. Katheter nach einem der Ansprüche 15–19, dadurch gekennzeichnet, dass er ein bleibend verformbares Element mit geringer Elastizität aufweist und im wesentlichen gerade vorgefertigt ist.

## Claims

1. Flexible peritoneal catheter for permanent implantation in peritoneal dialysis, comprising a distal end which can be connected to a connector, a region which can be passed through the abdominal wall of the patient and a proximal end, which can be introduced into the peritoneal cavity, and at least one bend, characterized in that the bend(s) is or are prefabricated stress-free in the catheter region which is fitted and/or implanted in front of and/or in the abdominal wall, in such a way that the bend(s) is or are convertible into the extended state during implantation and, due to its or their flexible properties, automatically returns or return after implantation to the prefabricated bent state.

2. Catheter according to Claim 1, characterized by two shanks (32; 34) which are located within the abdominal wall and are arranged substantially at right angles to one another.

3. Catheter according to Claim 1 or 2, characterized in that the region, located immediately before the abdominal wall, of the catheter (10, 53, 64) is bent off substantially at a right angle.

4. Catheter according to one of Claims 1–3, characterized in that the region (38), arranged before the peritoneal wall, of the catheter (10, 64) is bent off substantially at a right angle.

5. Catheter according to one of Claims 1–4, characterized in that the shanks (32, 34) have substantially the same length.

6. Catheter according to Claims 1–3 or 5, characterized by a plurality of wavy bends (68) in the region passing through the abdominal wall.

7. Catheter according to one of Claims 1–6, characterized by at least one disc (46, 48) which surrounds it and consists of a material compatible with the body.

8. Catheter according to Claim 7, characterized in that the disc (46, 48) has a sleeve (50, 52) which directly surrounds the catheter (10, 53, 64).

9. Catheter according to Claim 7 or 8, characterized in that the disc (46, 48) has a core region which is coated with a material compatible with the body, the materials of the core region and the sleeve (50, 52) being identical.

10. Catheter according to one of Claims 7–9, characterized in that the material compatible with the body is a polyester fabric based on polyethylene terephthalate (Dacron) and/or is titanium.

11. Catheter according ot one of Claims 7–10, characterized in that it has two discs (46, 48).

12. Catheter according to one of Claims 7–11, characterized in that it has one disc (46, 48) and one sleeve (58, 62).

13. Catheter according to one of Claims 1–12, characterized in that it consists of a silicone material.

14. Catheter according to one of Claims 1–13, characterized in that it is all in one plane.

15. Catheter according to one of Claims 1–14, characterized in that it has at least one permanently deformable element in the wall (72).

16. Catheter according to Claim 15, characterized in that it has one or more wires running in the longitudinal direction of the catheter.

17. Catheter according to Claim 15 or 16, characterized in that the wire has the form of a spiral (74).

18. Catheter according to one of Claims 15–17, characterized in that the permanently deformable element is provided in that region of the catheter (7) which can be passed through the abdominal wall.

19. Catheter according to one of Claims 15–18, characterized in that it has a permanently deformable element of high elasticity and is prefabricated in the bent-off state.

20. Catheter according to one of Claims 15–19, characterized in that it has a permanently deformable element of low elasticity and is prefabricated in a substantially straight state.

## Revendications

1. Cathéter péritonéal flexible destiné à l'implantation à demeure pour la dialyse péritonéale, qui comprend une extrémité distale puvant être raccordée à un connecteur, une région pouvant être passée à travers la paroi abdominale du patient et une extrémité proximale pouvant être enfoncée dans la cavité abdominale, et qui présente au moins un coude, caractérisé en ce que le coude ou les coudes est ou sont logé(s) et préfabriqué(s) sans tensions résiduelles, dans la région du cathéter qui est logée et/ou implantée en amont et/ou à l'intérieur de la paroi abdominale, de telle manière que le coude ou les coudes puisse(nt) être mis à l'état étiré au cours de l'implantation et revienne(nt) spontanément à l'état coudé préfabriqué après l'implantation, grâce à sa ou leur propriété de flexibilité.

2. Cathéter selon la revendication 1, caractérisé par deux branches (32, 34) placées dans la paroi abdominale, qui sont disposées sensiblement perpendiculairement l'une à l'autre.

3. Cathéter selon la revendication 1 ou 2, caractérisé en ce que la région du cathéter (10, 53, 64) situées immédiatement en amont de la paroi abdominale est coudée sensiblement à angle droit.

4. Cathéter selon l'une des revendications 1 à 3, caractérisé en ce que la région (38) du cathéter (10, 64) disposée en amont de la paroi péritonéale est coudée sensiblement à angle droit.

5. Cathéter selon l'une des revendications 1 à 4, caractérisé en ce que les branches (32, 34) sont sensiblement de même longueur.

6. Cathéter selon l'une des revendications 1 à 3 et 5, caractérisé par une pluralité de coudes (68) de forme ondulée présents dans la région qui travers la paroi abdominale.

7. Cathéter selon l'une des revendications 1 à 6, caractérisé par au moins une rondelle (46, 48) faite d'une matière physiologiquement compatible.

8. Cathéter selon la revendication 7, caractérisé en ce que la rondelle (46, 48) présente une manchette (50, 52) qui entoure directement le cathéter (10, 53, 64).

9. Cathéter selon la revendication 7 ou la revendication 8, caractérisé en ce que la rondelle (46, 48) possède une région noyau qui est revêtue d'une matière physiologiquement compatible, la matière de la région noyau et celle de la manchette (50, 52) étant identiques.

10. Cathéter selon l'une des revendications 7 à 9, caractérisé en ce que la matière physiologiquement compatible est un tissu de polyester à base de téréphthalate de polyéthylène «Dracon» et/ou le titane.

11. Cathéter selon l'une des revendications 7 à 10, caractérisé en ce qu'il comprend deux rondelles (46, 48).

12. Cathéter selon l'une des revendications 7 à 11, caractérisé en ce qu'il présente une rondelle (46, 48) et une manchette (58, 62).

13. Cathéter selon l'une des revendications 1 à 12, caractérisé en ce qu'il est fait d'une matière à base de silicone.

14. Cathéter selon l'une des revendications 1 à 13, caractérisé en ce qu'il est contenu dans un plan.

15. Cathéter selon l'une des revendications 1 à 14, caractérisé en ce qu'il comprend dans sa paroi (73) au moins un élément susceptible de déformation permanente.

16. Cathéter selon la revendication 15, caractérisé en ce qu'il comprend un ou plusieurs fil(s) métallique(s) s'étendant dans l'axe longitudinal du cathéter.

17. Cathéter selon la revendication 15 ou la revendication 16, caractérisé en ce que le fil métallique présente la forme d'une hélice (74).

18. Cathéter selon l'une des revendications 15 à 17, caractérisé en ce que l'élément susceptible de déformation permanente est prévu dans la région du cathéter (7) que l'on fait passer à travers la paroi abdominale.

19. Cathéter selon l'une des revendications 15 à 18, caractérisé en ce qu'il présente un élément susceptible de déformation permanente à haute élasticité et qu'il est préfabriqué avec une forme coudée.

20. Cathéter selon l'une des revendications 15 à 19, caractérisé en ce qu'il présente un élément susceptible de déformation permanente de faible élasticité et qu'il est préfabriqué sous une forme sensiblement rectiligne.

FIG 1

FIG 2

0081724

FIG 3

FIG 4

11

FIG5

FIG 6

FIG 7